# NEW EUROPEAN PATENT SPECIFICATION

(11) **EP 1 404 276 B2**
(45) Date of publication and mention of the opposition decision: **20.11.2013**
(45) Mention of the grant of the patent: 01.10.2008
(21) Application number: 02741728.6
(22) Date of filing: 24.05.2002
(51) Int. Cl.: A61F 13/47, A61F 13/84, A61F 13/472

(54) **LABIAL PAD HAVING A NOTCH**
LABIALE HYGIENEVORLAGE MIT EINER KERBE
COUSSINET LABIAL PRESENTANT UNE ENCOCHE

(30) Priority: 08.06.2001 US 297001 P; 27.08.2001 US 315255 P; 27.08.2001 US 315256 P; 31.12.2001 US 36635; 31.12.2001 US 36990
(43) Date of publication of application: 07.04.2004
(73) Proprietor: KIMBERLY-CLARK WORLDWIDE, INC., Neenah, WI 54956 (US)
(72) Inventor: EDENS, Ronald, L., Cumming, GA 30041 (US); HLABAN, James, J., Ogdensburg, Wisconsin 54962 (US); KEELY, Laura, J., Neenah, WI 54956 (US); KEENAN, Thomas, P., Appleton, WI 54911 (US); LITTLE, Sylvia, B., Marietta, GA 30062 (US); MCDANIEL, Mary, L., Appleton, WI 54911 (US); NUNN, Stephen, L., Appleton, WI 54914 (US); REEVES, William, G., Appleton, WI 54911 (US); SOREBO, Heather, A., Appleton, WI 54915 (US); WEYENBERG, Susan, M., Appleton, WI 54911 (US)
(74) Representative: Davies, Christopher Robert
(86) International application number: PCT/US2002/016477
(87) International publication number: WO 2002/100314

(56) References cited:
- EP-A- 0 162 451
- EP-A1- 0 162 451
- EP-A1- 0 888 765
- EP-A1- 1 407 746
- EP-A2- 0 605 017
- EP-A2- 0 613 671
- WO-A-00/40197
- WO-A-01/60297
- WO-A-98/29078
- WO-A1-00//40197
- WO-A1-01//60297
- WO-A1-98//29075
- WO-A1-98//29078
- WO-A1-98//57609
- US-A- 3 983 873
- US-A- 3 983 873
- US-A- 4 605 404
- US-A- 4 605 404
- US-A- 5 336 208
- US-A- 5 336 208
- US-A- 5 484 429
- US-A- 5 738 212
- US-A- 6 045 544

## Description

### BACKGROUND

The present invention relates generally to absorbent articles such as labial pads configured for disposition within the vestibule of a female wearer. More particularly, the present invention relates to labial pads having at least one notch situated on the periphery thereof.

A broad manner and wide variety of absorbent articles configured for the absorption of bodily exudates such as menstrual fluid are, of course, well known. With respect to feminine hygiene, the art has offered two basic types of feminine hygiene protection: sanitary napkins, developed for external wear about the pudendal region, and tampons, developed for residence within the vaginal cavity and interruption of menstrual flow therefrom. Hybrid feminine hygiene protection devices, attempting to merge the structural features of both within a single type of device, have also been proposed, but have not seen a meaningful measure of acceptance insofar as the effort to appropriate advantages has been overshadowed by the more demonstrable perpetuation of structural and anatomically functional disadvantages. Other less intrusive devices, known as labial or interlabial devices and characterized as having a portion which at least partially resides external of the wearer's vestibule, have also been proposed.

Many of these prior devices have not fully satisfied the demand of consumers for even smaller devices that may be worn interlabially by female wearers. In response thereto, several manufacturers have produced labial pads that are quite small in size in comparison to the prior devices described above. However, the construction of many of these devices appears to fail to recognize the significance of anatomical cooperation with the female wearer. For example, the obtrusive geometries of many of these devices result in structural elements coming into irritating contact with highly sensitive portions of the female anatomy such as the clitoris and/or the perineum.

### SUMMARY

The present inventors have recognized the deficiencies and problems inherent in the prior art and in response thereto conducted intensive research in developing innovative labial pads. The inventors have discovered that situating at least one notch in the periphery of at least that portion of an absorbent article, such as a labial pad, to be located nearest the clitoris minimizes the likelihood that the absorbent article will come into irritating contact with the clitoris. The inventors also discovered that situating at least one notch in the periphery of at least that portion of an absorbent article, such as a labial pad, to be located nearest the perineum minimizes the likelihood that the absorbent article will come into irritating contact with the perineum.

In one embodiment, an absorbent article is configured for disposition within the vestibule of a female wearer. The absorbent article includes an absorbent. The absorbent has a substantially circular geometry, a radius, a periphery and a central axis. The absorbent of this embodiment has at least one notch situated in its periphery.

In another alternative embodiment, an absorbent article is disclosed as being configured for disposition within the vestibule of a female wearer. The absorbent article includes a fluid permeable cover, a liquid impermeable baffle and an absorbent situated between the cover and the baffle. The absorbent has a substantially circular geometry, a radius, a periphery and a central axis. The absorbent also has at least one notch situated in its periphery.

In an additional alternative embodiment, an absorbent article is configured for disposition within the vestibule of a female wearer. The absorbent article includes a liquid impermeable baffle and an absorbent. The absorbent has a substantially circular geometry, a radius, a periphery and a central axis. The absorbent has at least one notch situated in its periphery.

In a further alternative embodiment, an absorbent article is disclosed as being configured for disposition within the vestibule of a female wearer. The absorbent article includes a fluid permeable cover and an absorbent. The absorbent has a substantially circular geometry, a radius, a periphery and a central axis. The absorbent has at least one notch situated in its periphery.

### DRAWINGS

The foregoing and other features, aspects and advantages of the present invention will become better understood with regard to the following description, appended claims and accompanying drawings where:
FIG. 1 is a simplified anatomical cross-sectional view of a human female illustrating the environment for an absorbent article such as a labial pad.
FIG. 2 is a simplified anatomical cross-sectional view of a human female illustrating the external genetalia.
FIG. 3 is a simplified view illustrating the positioning of an unfolded or substantially flat absorbent article having a notch located on that portion of the periphery thereof intended to be situated nearest the clitoris of a human female.
FIG. 4 is a top view illustrating a version of an absorbent article having at least one notch located on the periphery thereof.
FIG. 5 is cross-sectional view of the absorbent article illustrated in FIG. 4 taken along line 5 *-* 5 thereof.
FIG. 6 is a cross-sectional view illustrating another version of an absorbent article.
FIG. 7 is a top view illustrating an embodiment of a notch situated on the periphery of an absorbent article similar to that illustrated in FIG. 4.
FIG. 8 is a top view illustrating another embodiment of a notch situated on the periphery of yet another version of an absorbent article.
FIG. 9 is a top view illustrating yet another embodiment of a notch situated on the periphery of still another version of an absorbent article.
FIG. 10 is a top view illustrating still another embodiment of a notch situated on the periphery of a further version of an absorbent article.
FIG. 11 is a top view illustrating still a further version of an absorbent article having at least one notch situated along its periphery.
FIG. 12 is a cross-sectional view illustrating yet a further version of an absorbent article.
FIG. 13 is a cross-sectional view illustrating the version of FIG. 12 in a substantially folded position.
FIG. 14 illustrates an enlarged view of an additional version of an absorbent article folded substantially about an axis.
FIG. 15 illustrates an exaggerated enlarged view of the version of FIG. 14 folded substantially about an axis and being grasped for disposition in the vestibule by the wearer's fingers.
FIG. 16 is a top view of another additional version of an absorbent article having at least one notch situated along its periphery.
FIG. 17 is a top view of yet another additional version of an absorbent article having at least one notch situated along its periphery.
FIG. 18 is a top view of still another additional version of an absorbent article having at least one notch situated along its periphery.
FIG. 19 is a top view of an alternate version of an absorbent article having at least one notch situated along its periphery.
FIG. 20 is a top view of a further alternate version of an absorbent article having at least one notch situated along its periphery.
FIG. 21 is a top view of still another alternate version of an absorbent article having at least one notch situated along its periphery.
FIG. 22 is a simplified view illustrating the positioning of an unfolded or substantially flat absorbent article having a substantially circular geometry and a notch located on that portion of the periphery thereof intended to be situated nearest the clitoris of a human female.
FIG. 23 is a top view illustrating a version of an absorbent article having a substantially circular geometry and at least one notch located on the periphery thereof.
FIG. 24 is cross-sectional view of the absorbent article illustrated in FIG. 23 taken along line 24 - 24 thereof.
FIG. 25 is a top view illustrating an embodiment of a notch situated on the periphery of an absorbent article.
FIG. 26 is a top view illustrating another embodiment of a notch situated on the periphery of an absorbent article.
FIG. 27 is a top view illustrating yet another embodiment of a notch situated on the periphery of an absorbent article.
FIG. 28 is a top view illustrating a version of an absorbent article having at least one notch situated on the periphery thereof.
FIG. 29 is a partially broken top view illustrating an absorbent article having an alternate embodiment of a notch situated on the periphery thereof.
FIG. 30 is a partially broken top view illustrating an absorbent article having yet another alternate embodiment of a notch situated on the periphery thereof.
FIG. 31 is a partially broken top view illustrating an absorbent article having still another alternate embodiment of a notch situated on the periphery thereof.

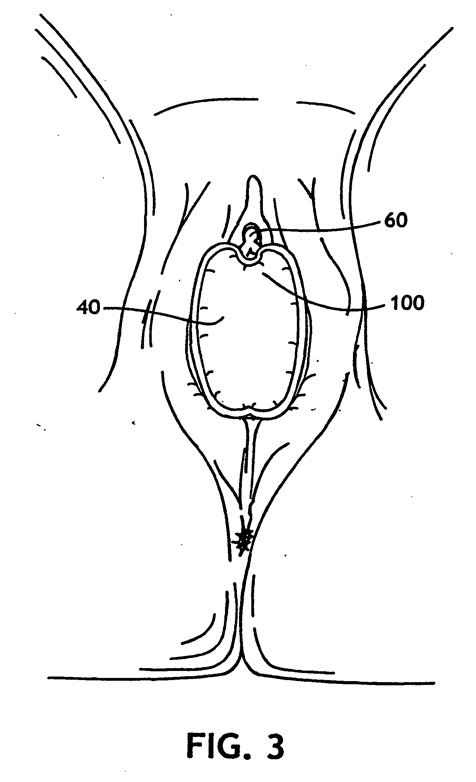

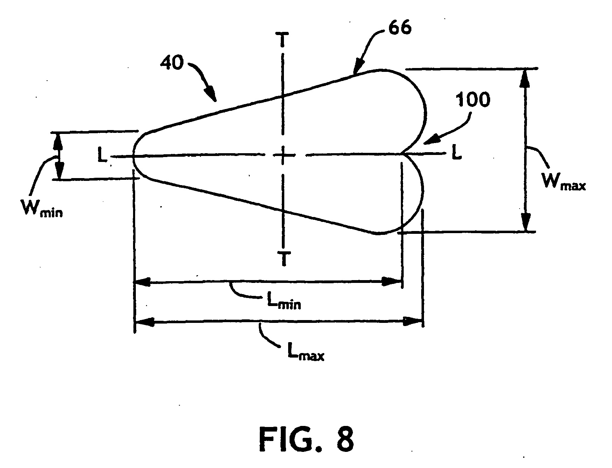

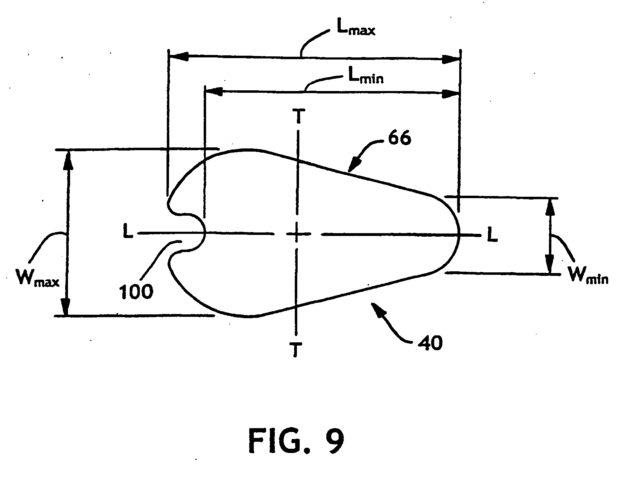

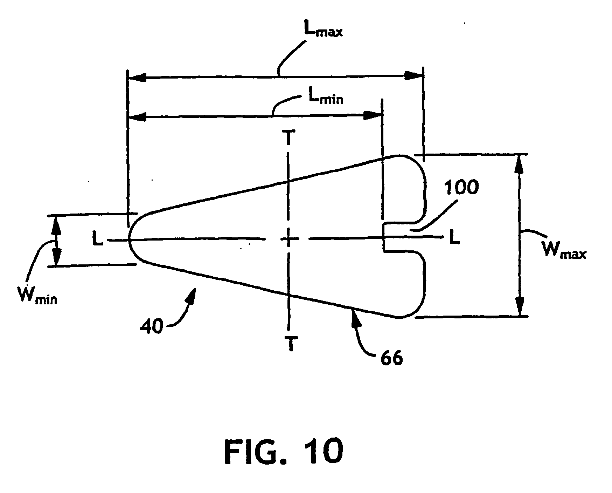

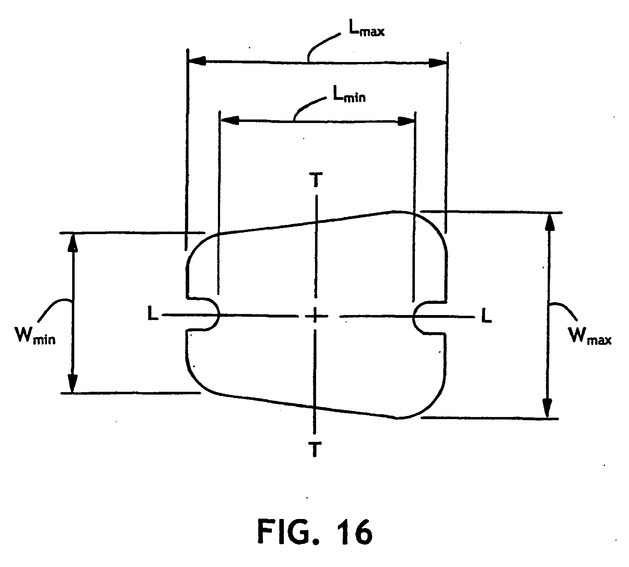

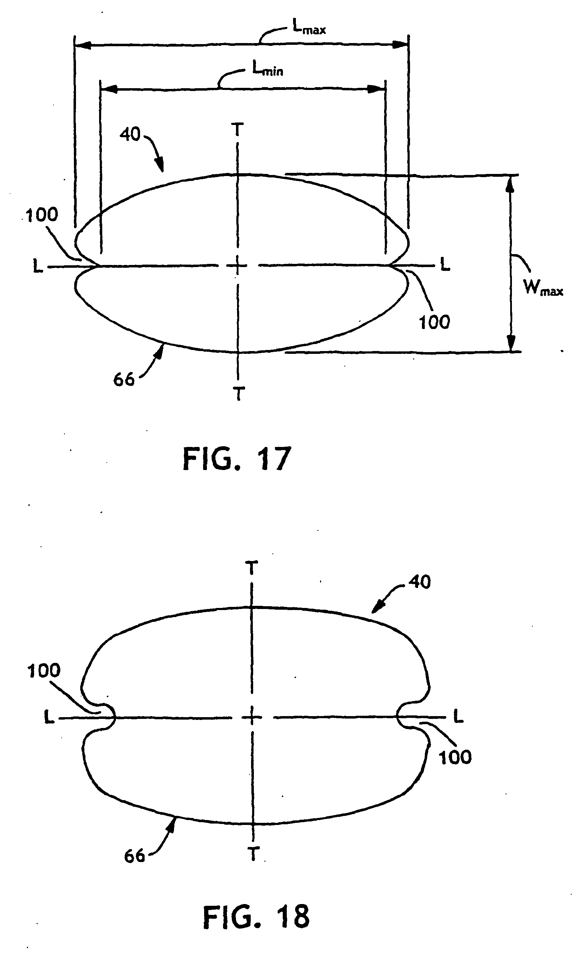

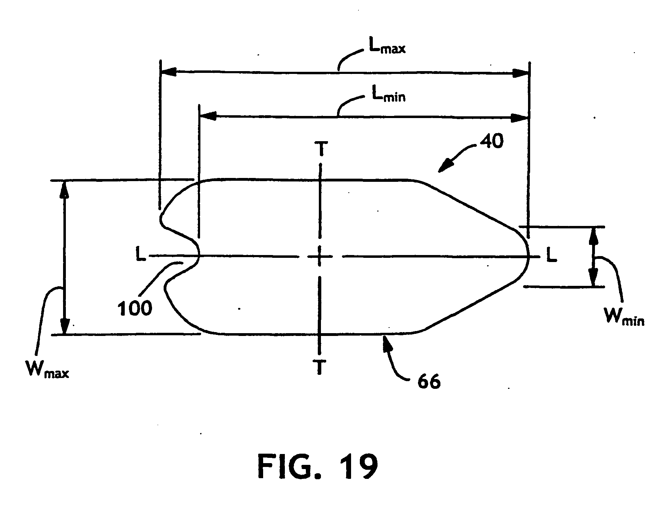

The embodiments described with reference to Figures 3 to 5, 7 to 11 and 16 to 21 do not call within the scope of the present invention.

### DESCRIPTION

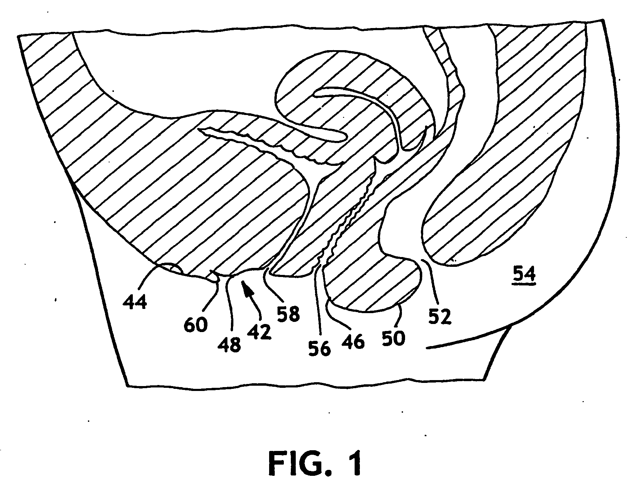

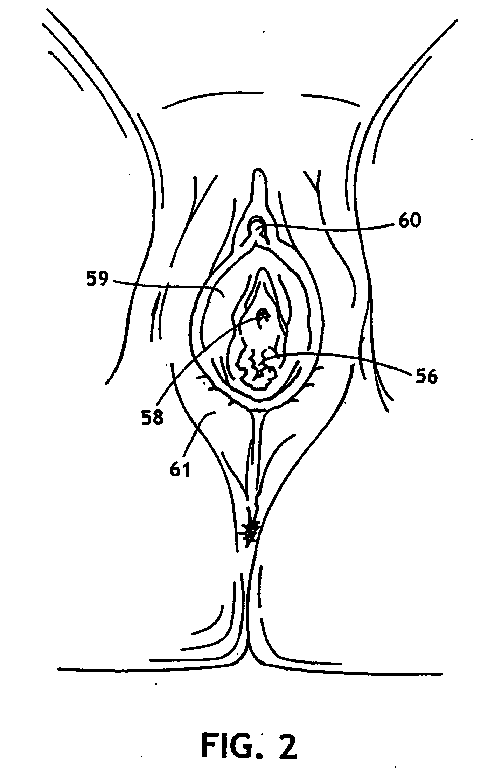

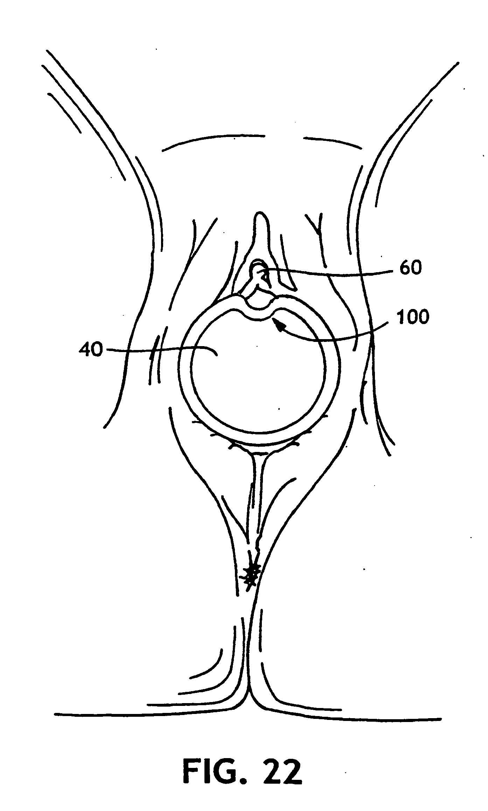

Turning to the figures of drawing, *i.e*., FIGs. 1 through 31, in each of which similar parts are identified with like reference characters, FIGs. 3 and 22 illustrate diagrammatically the positioning of an absorbent article, such as a labial pad, designated generally as 40, in an unfolded or flat configuration and prior to disposition within the vestibule of a wearer, designated generally as 42 (see also FIG. 1). As used herein, the term "labial pad" refers to a device having at least some absorbent components, and which is specifically configured for disposition in between the labia majora, extending at least partially into the vestibule (42) of a female wearer during use. For purposes of the ensuing description, the vestibule (42) is considered to be the region defined within the labia beginning at about a point lying caudally from the anterior labial commissure (44), extending rearward to the posterior labial commissure (46) and bounded inwardly by the floor (48) of the vestibule (see FIG. 1). One of skill in the art fully understands that there is a wide range of variation among women with respect to the relative size and shape of labia minora (59) and labia majora (61) as the same interrelatedly define the contour of the vestibule (42) (see FIGs. 1 and 2). For purposes of the present description, however, such differences will not specifically be addressed, it being recognized that in any event the disposition of the absorbent article (40) into the vestibule (42) will necessitate placement between the labia majora (61) regardless of any such consideration respecting the labia minora (59). Lying caudally of the vestibule (42) is the perineum (50) which leads to the anus (52) in the region of the buttocks (54). Within the vestibule (42) itself is located the principal urogenital members which, for purposes pertinent here, are constituted of the vaginal orifice (56), the urethral orifice (58), and the clitoris (60). Given the foregoing simplified review of this anatomical region, and to facilitate the present description, the vestibule (42) will be considered generally to be the region between the posterior labial commissure (46) and the clitoris (60), for convenience. For a more comprehensive description of this portion of the human female anatomy, however, attention is invited to Anatomy of the Human Body by Henry Gray, Thirtieth American Edition (Carmine D. Clemente ed., Lea & Febiger, 1985) at 1571-1581.

The absorbent article (40) discussed herein is intended to be disposed at least partially within the vestibule (42) for at least partially occluding the same respecting fluid flow therefrom. In this regard, the predominant use of the absorbent article (40) is for the absorption of menstrual fluid emitted via the vaginal orifice (56); although the absorbent article is equally well adapted to serve as a type of incontinence device for absorption of urine as occurs upon minor, female incontinence.

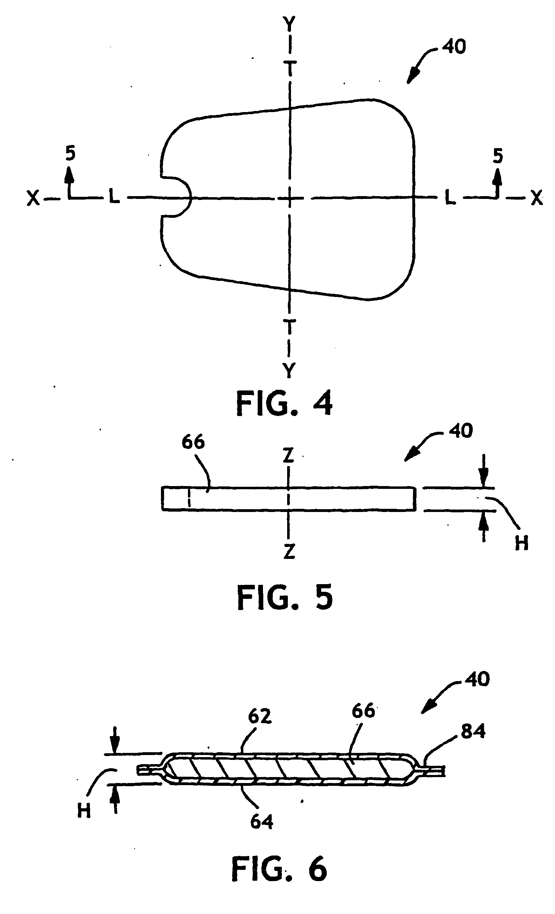

The absorbent article (40), a version of which is illustrated in FIG. 4, has a principal longitudinal axis (L) which generally runs along the x direction. As used herein, the term "longitudinal" refers to a line, axis or direction in the plane of the absorbent article (40) that is generally aligned with (*e.g.*, approximately parallel to) a vertical plane that bisects a standing female wearer into left and right body halves when the absorbent article is in use. The longitudinal direction is generally illustrated in FIG. 4 by the x-axis. The absorbent article (40) also has a principal transverse axis (T). The terms "trans verse," "lateral" or "y direction" as used herein generally refer to a line, axis or direction that is generally perpendicular to the longitudinal direction. The lateral direction is generally illustrated in FIG. 4 by the y-axis. The "z direction" is typically a line, axis or direction generally parallel to the vertical plane described above. The z direction is generally illustrated in FIG. 5 by the z-axis. The term "upper" refers generally to an orientation directed toward the wearer's head, while the terms "lower" or "downwardly" refer generally to an orientation directed toward the wearer's feet. For purposes of discussion herein, each layer of the absorbent article (40), *e.g*., a fluid permeable cover (62), a liquid impermeable baffle (64) and/or an absorbent (66), has an upper or body-facing surface and a lower surface also described as the surface opposed to the upper or body-facing surface.

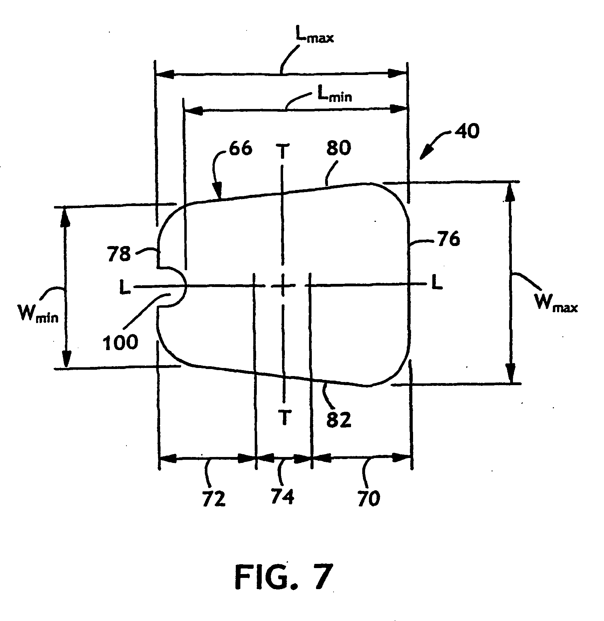

Turning now to FIG. 6, an absorbent article (40) is illustrated as including a fluid permeable cover (62), a liquid impermeable baffle (64) and an absorbent (66) situated between the cover and the baffle. As illustrated in FIG. 7, the absorbent (66) has a first end region (70), a second end region (72), and a central region (74) disposed between each end region. The absorbent article (40) should be of a suitable size and shape that allows at least a portion of the absorbent article to be disposed within the vestibule (42) of a female wearer. In addition, the absorbent article (40) desirably at least partially occludes and intercepts the flow of menstrual fluid, urine or other bodily exudates from the wearer's vaginal orifice (56) and/or urethral orifice (58).

The absorbent (66), and thus the absorbent article (40), generally displays a geometry extending between spaced apart first (76) and second (78) transverse end areas. The overall geometry is completed by noting that the absorbent (66), and thus the absorbent article (40), also includes spaced apart first (80) and second (82) longitudinal sides ranging between the transverse end areas (76, 78), these collectively sometimes being referred to herein as the perimetral sides (*i.e*., those defining the periphery).

The geometry of the absorbent (66) is a significant factor affecting the overall size and effectiveness of the absorbent article (40). In general, the absorbent (66) has a maximum width (Wₘₐₓ), measured along a line laying generally parallel to the principal transverse axis (T) and running from one longitudinal side to the opposing longitudinal side (80, 82), and a minimum width (Wₘᵢₙ), measured along a line also laying generally parallel to the principal transverse axis (T) and running from one longitudinal side to the opposing longitudinal side (80, 82). The maximum width (Wₘₐₓ) of the absorbent (66) typically is no greater than about 30; alternatively, no greater than about 40; alternatively, no greater than about 50; alternatively, no greater than about 60; or alternatively, no greater than about 70 mm. The minimum width (Wₘᵢₙ) of the absorbent (66) typically is no less than about 30; alternatively, no less than about 20; alternatively, no less than about 10; or alternatively, no less than about 5 mm. Thus, the absorbent (66) may have a width ranging between no less than about 5 mm up to no greater than about 70 mm; although the approximate width(s) of the absorbent may vary according to, *inter alia*, the general design and intended disposition of the absorbent article (40) within the vestibule (42) of a female wearer. One of skill in the art will readily appreciate that certain versions of the absorbent (66), and thus certain versions of the absorbent article (40), may have a minimum width (Wₘᵢₙ) equal to its maximum width (Wₘₐₓ). In such instances, reference is generally made only to the maximum width (Wₘₐₓ).

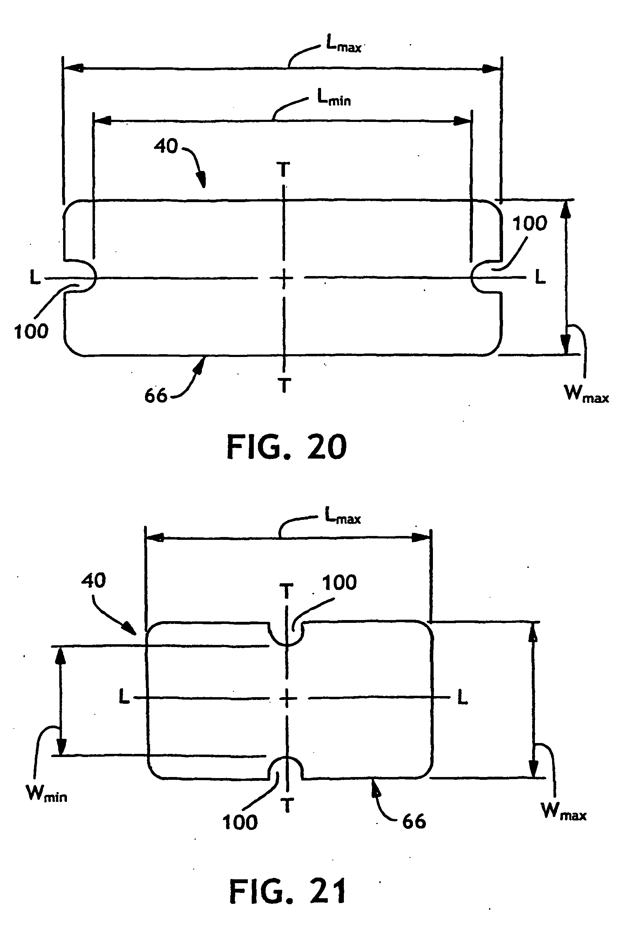

The absorbent (66) also has a maximum length (Lₘₐₓ), measured along a line laying generally parallel to the principal longitudinal axis (L) and running from one transverse end area to the other transverse end area (76, 78). The maximum length (Lₘₐₓ) of the absorbent (66) typically is no greater than about 40; alternatively, no greater than about 50; alternatively, no greater than about 60; alternatively, no greater than about 70; alternatively, no greater than about 80; alternatively, no greater than about 90; or alternatively, no greater than about 100 mm. The absorbent (66) may also have a minimum length (Lₘᵢₙ), measured along a line also laying generally parallel to the principal longitudinal axis (L) and running from one transverse end area to the other transverse end area (76, 78). The minimum length (Lₘᵢₙ) of the absorbent (66) typically is no less than about 100; alternatively, no less than about 90; alternatively, no less than about 80; alternatively, no less than about 70; alternatively, no less than about 60; alternatively, no less than about 50; or alternatively, no less than about 40 mm. Thus, the absorbent (66) may have a length ranging between no less than about 40 mm up to no greater than about 100 mm; although the approximate length(s) of the absorbent may vary according to, *inter alia*, the general design and intended disposition of the absorbent article (40) within the vestibule (42) of a female wearer. One of skill in the art will readily appreciate that certain versions of the absorbent (66), and thus certain versions of the absorbent article (40), may have a minimum length (Lₘᵢₙ) equal to its maximum length (Lₘₐₓ₎. In such instances, as illustrated at least in FIG. 21, reference is generally made only to the maximum length (Lₘₐₓ). Versions of an absorbent (66), and thus versions of an absorbent article (40), having a maximum, length (Lₘₐₓ) not equal to its minimum length (Lₘᵢₙ) are illustrated at least in FIGs. 7 through 11 and 16 through 20.

The first end region (70) and the second end region (72) each minimally extend outwardly from the central region (74) toward the transverse end areas (76 and 78, respectively) of the absorbent (66) a distance of no less than about 30; alternatively, no less than about 20; or alternatively, no less than about 10 % of the maximum length (Lₘₐₓ) of the absorbent. The first end region (70) and the second end region (72) each maximally extend outwardly from the central region (74) toward the transverse end areas (76 and 78, respectively) of the absorbent (66) a distance of no greater than about 20; alternatively, no greater than about 30; or alternatively, no greater than about 40 % of the maximum length (Lₘₐₓ) of the absorbent. Thus, the end regions (70, 72) may occupy from a minimum of about 20 % up to a maximum of about 80 % of the maximum length (Lₘₐₓ) of the absorbent (66); although the approximate size of the first and second end regions may vary according to, *inter alia*, the general design and intended disposition of the absorbent article (40) within the vestibule (42) of a female wearer.

The absorbent article (40) is desirably provided with sufficient capacity to absorb and retain the intended amount and type of bodily exudate(s). The absorbent capacity is provided by a fluid retentive core or absorbent generally identified as 66. For at least menstrual fluid, the absorbent (66) desirably has a minimum capacity of no less than about 19; alternatively, no less than about 18; alternatively, no less than about 17; alternatively, no less than about 16; alternatively, no less than about 15; alternatively, no less than about 14; alternatively, no less than about 13; alternatively, no less than about 12; alternatively, no less than about 11; alternatively, no less than about 10; alternatively, no less than about 9; alternatively, no less than about 8; alternatively, no less than about 7; alternatively, no less than about 6; alternatively, no less than about 5; alternatively, no less than about 4; alternatively, no less than about 3; alternatively, no less than about 2; or alternatively, no less than about 1 g/g. The absorbent (66) also may have a maximum capacity of no greater than about 5; alternatively, no greater than about 6; alternatively, no greater than about 7; alternatively, no greater than about 8; alternatively, no greater than about 9; alternatively, no greater than about 10; alternatively, no greater than about 11; alternatively, no greater than about 12; alternatively, no greater than about 13; alternatively, no greater than about 14; alternatively, no greater than about 15; alternatively, no greater than about 16; alternatively, no greater than about 17; alternatively, no greater than about 18; alternatively, no greater than about 19; alternatively, no greater than about 20; alternatively, no greater than about 25; or alternatively, no greater than about 30 g/g. Thus, the absorbent (66) may have an absorbent capacity ranging between no less than about 1 g/g up to no greater than about 30 g/g; although the approximate capacity of the absorbent may vary according to, *inter alia*, the general design and intended disposition of the absorbent article (40) within the vestibule (42) of a female wearer. One of skill in the art will readily realize that the addition of superabsorbent polymer(s) or coated superabsorbent polymer(s) to the absorbent (66) typically has the effect of substantially increasing the absorbent capacity.

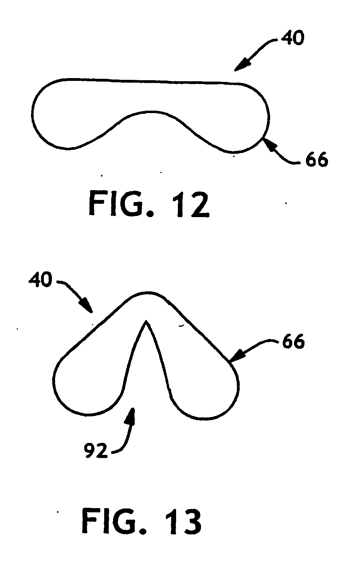

Describing the individual elements in greater detail, the absorbent (66) has an upper or body-facing surface and a lower surface (or surface opposed to the upper or body-facing surface) and may include any material capable of absorbing and/or adsorbing and thereafter retaining the intended bodily exudate(s). Suitable materials are also generally hydrophilic, compressible and conformable. The absorbent (66) may be formed from any of the materials well known to those of ordinary skill in the art. Examples of such materials include, but are not limited to, various natural or synthetic fibers, multiple plies of creped cellulose wadding, fluffed cellulose fibers, rayon or other regenerated cellulose materials, wood pulp fibers or comminuted wood pulp fibers, airlaid material, textile fibers, a blend of polyester and polypropylene fibers, absorbent foams, absorbent sponges, superabsorbent polymers, coated superabsorbent polymers, fibrous bundles or nits, or any equivalent material or combination of materials. Also suitable for use would be hydrophobic material that has been rendered hydrophilic according to any of a number of known methods for so doing. The total absorbent capacity of the absorbent (66) should, however, be compatible with the design exudate loading and the intended use of the absorbent article (40). Further, the size and absorbent capacity of the absorbent (66) may be varied. Therefore, the dimension, shape, and configuration of the absorbent (66) may be varied (*e.g*., the absorbent may have a varying thickness as illustrated at least in FIGs. 12 and 13, or a hydrophilic gradient, or may contain superabsorbent polymer(s) and the like).

The absorbent (66) generally has a thickness, caliper or height (H), as illustrated at least in FIG. 5, measured along a line lying generally parallel to the z-axis. The minimum thickness of the absorbent (66) typically is no less than about 9; alternatively, no less than about 8; alternatively, no less than about 7; alternatively, no less than about 6; alternatively, no less than about 5; alternatively, no less than about 4; alternatively, no less than about 3; alternatively, no less than about 2; alternatively, no less than about 1; or alternatively, no less than about 0.5 mm. The maximum thickness of the absorbent (66) typically is no greater than about 2; alternatively, no greater than about 3; alternatively, no greater than about 4; alternatively, no greater than about 5; alternatively, no greater than about 6; alternatively, no greater than about 7; alternatively, no greater than about 8; alternatively, no greater than about 9; or alternatively, no greater than about 10 mm. Thus, the absorbent (66) may have a thickness of about 10 mm or less; although the approximate thickness of the absorbent may vary according to, *inter alia*, the general design and intended disposition of the absorbent article (40) within the vestibule (42) of a female wearer.

The absorbent (66) desirably also has a relatively low density which is deemed desirable for comfort. Generally, the absorbent has a density of less than about 0.5 g/cc. Stated differently, the absorbent (66) typically has a maximum density of no greater than about 0.5; alternatively, no greater than about 0.4; alternatively, no greater than about 0.3; alternatively, no greater than about 0.2; alternatively, no greater than about 0.1; alternatively, no greater than about 0.09; alternatively, no greater than about 0.08; alternatively, no greater than about 0.07; alternatively, no greater than about 0.06; alternatively, no greater than about 0.05; alternatively, no greater than about 0.04; alternatively, no greater than about 0.03; or alternatively, no greater than about 0.02 g/cc. The absorbent (66) generally also has a minimum density of typically no less than about 0.01; alternatively no less than about 0.02; alternatively, no less than about 0.03; alternatively, no less than about 0.04; alternatively, no less than about 0.05; alternatively, no less than about 0.06; alternatively, no less than about 0.07; alternatively, no less than about 0.08; alternatively, no less than about 0.09; alternatively, no less than about 0.1; alternatively, no less than about 0.2; alternatively, no less than about 0.3; or alternatively, no less than about 0.4 g/cc. Thus, the density of the absorbent (66) may range up to about 0.5 g/cc; although the approximate density of the absorbent may vary according to, *inter alia*, the general design and intended disposition of the absorbent article (40) within the vestibule (42) of a female wearer.

The absorbent (66) also desirably has a basis weight of less than about 600 grams per square meter (gsm). Stated differently, the absorbent (66) typically has a maximum basis weight of no greater than about 600; alternatively, no greater than about 500; alternatively, no greater than about 400; alternatively, no greater than about 300; alternatively, no greater than about 200; or alternatively, no greater than about 100 gsm. Generally, the absorbent (66) also has a minimum basis weight of typically no less than about 0.1; alternatively, no less than about 50; alternatively, no less than about 100; alternatively, no less than about 150; alternatively, no less than about 200; alternatively, no less than about 250; alternatively, no less than about 300; alternatively, no less than about 350; alternatively, no less than about 400; alternatively, no less than about 450; alternatively, no less than about 500; or alternatively, no less than about 550 gsm. Thus, the absorbent (66) may have a basis weight of about 600 gsm or less; although the approximate basis weight of the absorbent may vary according to, *inter alia*, the general design and intended disposition of the absorbent article (40) within the vestibule (42) of a female wearer. A specific example of a suitable absorbent would be similar to a coform material made of a blend of polypropylene and cellulose fibers and used in KOTEX^{®} maxi pantiliners and obtainable from Kimberly-Clark Corporation, Neenah, WI, USA.

The optional baffle (64) typically resides on the lower surface of the absorbent (66) and may be constructed from any desired material that is liquid-impermeable. Desirably, the baffle (64) will permit the passage of air and moisture vapor out of the absorbent (66), while blocking the passage of bodily fluid(s). An example of a suitable baffle material is a micro-embossed, polymeric film, such as polyethylene, polypropylene or polyester, having a minimum thickness of no less than about 0.025 mm and a maximum thickness of no greater than about 0.13 mm. Bicomponent films can also be used, as well as woven and nonwoven fabrics which have been treated to render them liquid-impermeable. An example of another suitable material is a closed cell polyolefin foam. A closed cell polyethylene foam may also work well.

The baffle (64) may be maintained in secured relation with the absorbent (66) by bonding all or a portion of the adjacent surfaces to one another. A variety of bonding methods known to one of skill in the art may be utilized to achieve any such secured relation. Examples of such methods include, but are not limited to, ultrasonics, thermal bonding, or the application of adhesives in a variety of patterns between the two adjoining surfaces. A specific example of a baffle material would be similar to a polyethylene film used on KOTEX^{®} pantiliners and obtainable from Pliant Corporation, Schaumburg, IL, USA.

The optional fluid permeable cover (62) has an upper surface and a lower surface, with the upper surface typically contacting the body of the wearer and receiving bodily exudate(s). The cover (62) desirably is made of a material that is flexible and non-irritating to the tissues within the vestibule (42) of a female wearer. As used herein, the term "flexible" is intended to refer to materials which are compliant and readily conform to the bodily surface(s) or respond by easily deforming in the presence of external forces.

The cover (62) is provided for comfort and conformability and functions to direct bodily exudate(s) away from the body and toward the absorbent (66). The cover (62) should retain little or no liquid in its structure so that it provides a relatively comfortable and non-irritating surface next to the tissues within the vestibule (42) of a female wearer. The cover (62) can be constructed of any woven or nonwoven material which is also easily penetrated by bodily fluids contacting its surface. Examples of suitable materials include rayon, bonded carded webs of polyester, polypropylene, polyethylene, nylon, or other heat-bondable fibers, polyolefins, such as copolymers of polypropylene and polyethylene, linear low-density polyethylene, aliphatic esters such as polylactic acid, finely perforated film webs and net material also work well. A specific example of a suitable cover material would be similar to a bonded carded web made of polypropylene and polyethylene used as a cover stock for KOTEX^{®} pantiliners and obtainable from Sandler Corporation, Germany. Other examples of suitable materials are composite materials of a polymer and a nonwoven fabric material. The composite materials are typically in the form of integral sheets generally formed by the extrusion of a polymer onto a web of spunbond material. The fluid permeable cover (62) can also contain a plurality of apertures (not shown) formed therein which are intended to increase the rate at which bodily fluid(s) can penetrate into the absorbent (66).

A physiologically hydrous cover material is also suitable for use. As used herein, the term "phys iologically hydrous" is intended to connote a cover material which maintains a suitably moist interface between the tissues of the vestibule (42) and the absorbent article (40) when disposed in that vestibular environment; one that is benign respecting the requirements of comfort associated with the interposition of fabric or fabric-like structures within the moist tissue environment of the vestibule, keeping in mind as well the self-evident factor that the absorbent article is receiving bodily fluid(s) migrating through the vestibule and must conduct the same to the absorbent (66). Thus, while not "hydrous " in the classic sense prior to use (inasmuch as the cover will be dry at that time) the cover (62) maintains (or at least does not interfere with the maintenance of) the proper moisture level or balance required within the vestibule (42).

The cover (62) can also have at least a portion of the surface treated with a surfactant to render the cover more hydrophilic. This results in permitting the insulting bodily fluid(s) to more readily penetrate the cover (62). The surfactant may also diminish the likelihood that the insulting bodily fluid(s), such as menstrual fluid, will flow off the cover (62) rather than being absorbed by the absorbent (66). One suitable approach provides for the surfactant to be substantially evenly distributed across at least a portion of the upper surface of the cover (62) that overlays the upper surface of the absorbent (66).

The cover (62) may be maintained in secured relation with the absorbent (66) by bonding all or a portion of the adjacent surfaces to one another. A variety of bonding methods known to one of skill in the art may be utilized to achieve any such secured relation. Examples of such methods include, but are not limited to, the application of adhesives in a variety of patterns between the two adjoining surfaces, entangling at least portions of the adjacent surface of the absorbent with portions of the adjacent surface of the cover, or fusing at least portions of the adjacent surface of the cover to portions of the adjacent surface of the absorbent.

The cover (62) typically resides on the upper surface of the absorbent (66), but alternatively can surround and partially or entirely enclose the absorbent. Alternatively, the cover (62) and the baffle (64) can have peripheries which extend outward beyond the periphery of the absorbent (66) and can be peripherally joined together to form an edge (84), as illustrated at least in FIG. 6. Utilizing known techniques, such as, for example, gluing, crimping, hot-sealing or the like, the edge (84) may be formed either entirely, so that the entire periphery of the absorbent (66) is circumscribed by their joinder, or the cover (62) and the baffle (64) can be partially peripherally joined. To minimize the possibility of irritation and/or discomfort to the wearer of the absorbent article (40), it is desired that the edge (84) and at least the area of the absorbent article immediately adjacent the edge be soft, compressible and conformable. Desirably, any edge (84) so formed shall have a width no greater than about 10; alternatively, no greater than about 9; alternatively, no greater than about 8; alternatively, no greater than about 7; alternatively, no greater than about 6; alternatively, no greater than about 5; alternatively, no greater than about 4; alternatively, no greater than about 3; alternatively, no greater than about 2; or alternatively, no greater than about 1 mm. In addition, any edge (84) so formed shall desirably have a width of no less than about 0.5; alternatively, no less than about 1; alternatively, no less than about 2; alternatively, no less than about 3; alternatively, no less than about 4; alternatively, no less than about 5; alternatively, no less than about 6; alternatively, no less than about 7; alternatively, no less than about 8; or alternatively, no less than about 9 mm. Thus, any edge (84) so formed may have a width ranging from no less than about 0.5 mm up to no greater than about 10 mm; although the approximate width of any edge may vary according to, *inter alia*, the general design and intended disposition of the absorbent article (40) within the vestibule (42) of a female wearer. In other embodiments, the cover (62) and/or the baffle (64) can have a periphery that is coterminous with the periphery of the absorbent (66).

Positioned either on or substantially parallel to the principal longitudinal axis (L) of certain versions of the absorbent (66), is, optionally, a desired axis of flexure (F). A desired axis of flexure (F) generally runs in the longitudinal direction, *i.e*., along the x direction, and may be off center from the principal longitudinal axis (L) a distance of no greater than about 10; alternatively, no greater than about 9; alternatively, no greater than about 8; alternatively, no greater than about 7; alternatively, no greater than about 6; alternatively, no greater than about 5; alternatively, no greater than about 4; alternatively, no greater than about 3; alternatively, no greater than about 2; or alternatively, no greater than about 1 mm. Desirably, a desired axis of flexure (F) is aligned along the principal longitudinal axis (L). A desired axis of flexure (F) typically minimally extends longitudinally no less than about 90; alternatively, no less than about 80; alternatively, no less than about 70; alternatively, no less than about 60; alternatively, no less than about 50; or alternatively, no less than about 40 % of the maximum length (Lₘₐₓ) of the absorbent (66). A desired axis of flexure (F) typically extends longitudinally no greater than about 50; alternatively, no greater than about 60; alternatively, no greater than about 70; alternatively, no greater than about 80; alternatively, no greater than about 90; or alternatively, no greater than about 100 % of the maximum length (Lₘₐₓ) of the absorbent (66).

A desired axis of flexure (F) may result naturally from the dimensions, shape, and/or configuration of the absorbent (66), or the absorbent may be imparted with a weakened axis or region to create a desired axis of flexure. A desired axis of flexure (F) may also be formed by any of the techniques known to one of skill in the art, including, for example, scoring, pre-folding, slitting, embossing, or the like. Although a desired axis of flexure (F) is described herein as residing in the absorbent (66), one of skill in the art will readily appreciate that a desired axis of flexure may be formed in either the cover (62), the baffle (64) and/or the absorbent; the cover and the baffle; the cover and the absorbent; or the baffle and the absorbent. When present, a desired axis of flexure (F) typically allows an absorbent article (40) to be folded more easily prior to disposition within the vestibule (42) of a female wearer.

The absorbent article (40) also has a thickness, caliper or height (H), as illustrated at least in FIGs. 5 and 6, measured along a line laying generally parallel to the z-axis. The minimum thickness of the absorbent article (40) typically is no less than about 9; alternatively, no less than about 8; alternatively, no less than about 7; alternatively, no less than about 6; alternatively, no less than about 5; alternatively, no less than about 4; alternatively, no less than about 3; alternatively, no less than about 2; alternatively, no less than about 1; or alternatively, no less than about 0.5 mm. The maximum thickness of the absorbent article (40) typically is no greater than about 1; alternatively, no greater than about 2; alternatively, no greater than about 3; alternatively, no greater than about 4; alternatively, no greater than about 5; alternatively, no greater than about 6; alternatively, no greater than about 7; alternatively, no greater than about 8; alternatively, no greater than about 9; or alternatively, no greater than about 10 mm. Thus, the absorbent article (40) may have a thickness of about 10 mm or less; although the approximate thickness of the absorbent article may vary according to, *inter alia*, the general design and intended disposition of the absorbent article within the vestibule (42) of a female wearer.

The absorbent article (40) typically is folded along an axis, as illustrated at least in FIGs. 13, 14 and 15, prior to disposition within the vestibule (42) of the female wearer. When folded along such an axis, the absorbent article (40) will form a recess (92) which protects the wearer's finger(s) from soiling when the absorbent article is disposed within the vestibule (42). Once inserted, the absorbent article (40) may have a tendency to unfold in an attempt to fill the vestibule and thus maintain the upper surface of the absorbent article in contact with the tissues of the vestibule (42). The absorbent article (40) may be resiliently biased along the axis about which it is folded to increase the tendency of the absorbent article to unfold. Alternatively, the absorbent (66) of the absorbent article (40) may be thicker along its longitudinal edges, as illustrated at least in FIGs. 12 and 13, thus also demonstrating a biasing effect, if desired, which is typically intended to allow the upper surface of the absorbent article (40) to contact the tissues of the vestibule (42). An absorbent article (40) as described herein, however, does not necessarily require any additional features to maintain contact with the tissues of the vestibule (42) of the female wearer. The naturally moist surfaces of the tissues of the vestibule (42) typically demonstrate a tendency to maintain contact with the upper surface of the absorbent article (40).

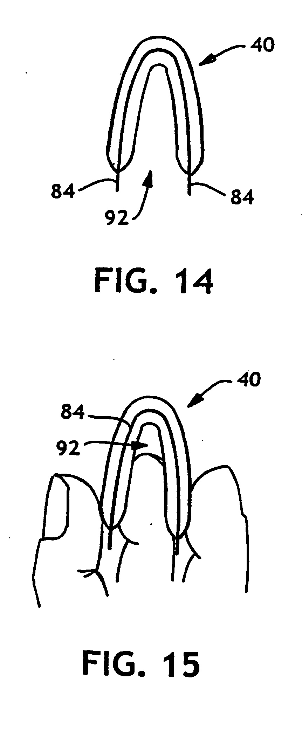

As noted above, the wearer may fold the absorbent article (40) along an axis prior to disposition within the vestibule (42). The wearer may, therefore, hold the folded absorbent article (40) at the periphery as illustrated at least in FIG. 15. The absorbent article (40) may then be disposed within the vestibule (42) by the wearer exerting a force with a finger or fingers positioned in the recess (92) formed by the folded absorbent article.

As illustrated at least in FIGs. 3, 4, 7 through 11 and 16 through 21, the absorbent (66), and thus an absorbent article (40), is provided with at least one notch (100) expending inward from the periphery. As used herein, The term "notch" refers to a space, indentation or hollow along the periphery of a material, layer of material or laminate of materials. Because of the numerous possible geometries for the absorbent (66), and thus the absorbent article (40), it is almost impossible to indicate where on a particularly configured absorbent article the notch (100) should be located without seeing that particular absorbent article in use. However, it has been determined that when located at least in the periphery of that portion of the absorbent article (40) to be situated nearest the clitoris (60), the notch (100) maximizes the possibility that the absorbent article will maintain a substantially spaced relationship from a female wearer's clitoris when the absorbent article is disposed in a female wearer's vestibule (42). Such a spaced relationship is believed to minimize the likelihood that the absorbent article (40) will contact the sensitive clitoris (60), thus guarding against the irritating and perhaps painful chafing effects which contact by an absorbent article with the clitoris can occasion.

It has also been determined that when located at least in the periphery of that portion of the absorbent article (40) to be situated nearest the perineum (50), the notch (100) minimizes the likelihood that the absorbent article will come into irritating contact with the sensitive perineal region. This is believed to be significant for those wearers who would use the absorbent article (40) post-partum when the perineal region is highly sensitized or has been sewn due to tearing or having been cut during childbirth. It is noteworthy, however, that even those wearers who are nulliparous, *i.e*., the perineal region has not been exposed or experienced stretching, tearing or cutting during childbirth, may also have highly sensitive perineal regions.

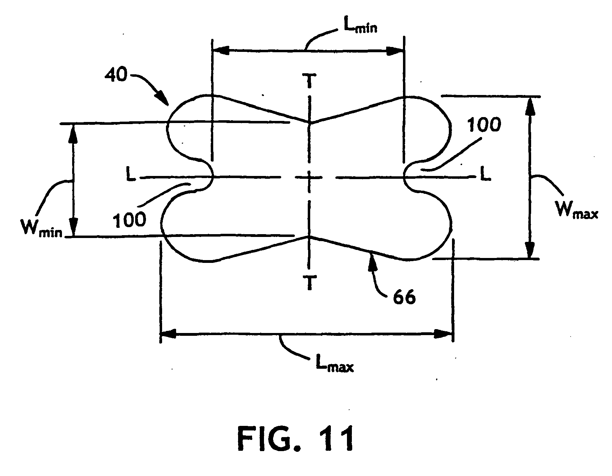

Specifically, an absorbent (66), and thus an absorbent article (40), may include at least one notch (100) extending inward from the periphery of at least one of the transverse end areas (76, 78). The notch (100) may, for example, be situated substantially on or adjacent to the principal longitudinal axis (L) of the absorbent (66). Alternatively, the notch (100) may be situated substantially on or adjacent to a desired axis of flexure (F). The notch (100) may also provide a natural folding or bending line to the absorbent (66) thus allowing the absorbent article, when folded or bent along any such line, to be more easily folded or bent. This is believed to be particularly true when an absorbent (66), as illustrated at least in FIGs. 11, 16 through 18 and 20, has at least one notch (100) situated in the periphery of each opposing transverse end area (76, 78). When the absorbent article (40) is disposed within the vestibule, the notch (100), when located at least in the periphery of the transverse end area to be situated nearest the clitoris (60), minimizes the likelihood that the absorbent article (40) will irritatingly contact the sensitive clitoris.

In another version, an absorbent (66), and thus an absorbent article (40), includes at least one notch (100) extending inward from the periphery of at least one of the longitudinal sides (80, 82). The notch (100) may, for example, be situated substantially on or adjacent to the principal transverse axis (T) of the absorbent (66). The notch (100) may also provide a natural folding or bending line to the absorbent (66) thus allowing the absorbent article, when folded or bent along any such transverse axis or line, to be more easily folded or bent. This is believed to be particularly true when an absorbent (66), a version of which is illustrated at least in FIG. 21, has at least one notch (100) situated in the periphery of each opposing longitudinal side (80, 82). When the absorbent article (40) is disposed within the vestibule, the notch (100), when located at least in the periphery of the longitudinal side to be situated nearest the clitoris (60), minimizes the likelihood that the absorbent article (40) will irritatingly contact the sensitive clitoris.

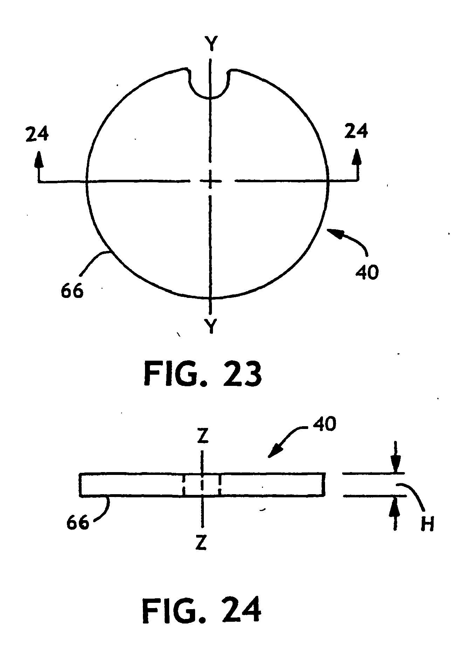

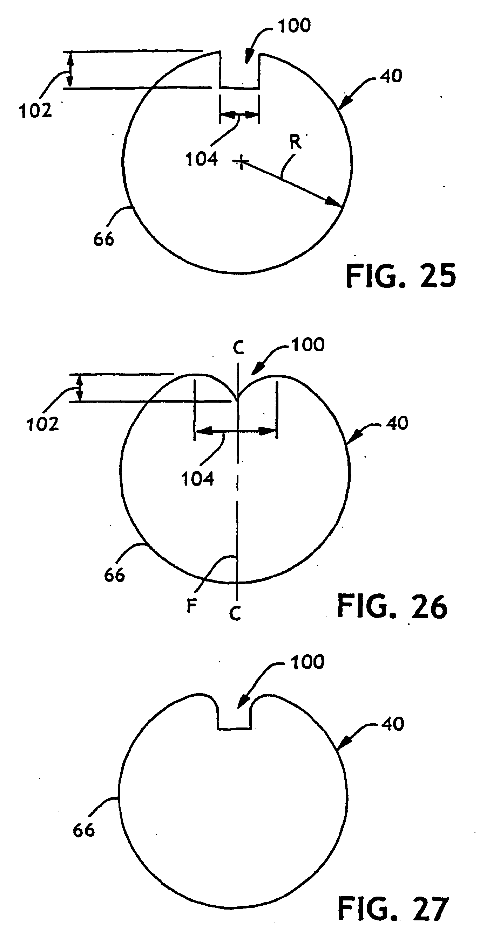

In still another version, as illustrated in FIG. 26, an absorbent (66), and thus an absorbent article (40), has a central axis (C) which generally bisects the absorbent article into substantially identical halves. The absorbent (66), and thus the absorbent article (40), generally displays a substantially circular geometry as illustrated at least in FIGs. 23, 25, 26 and 27. When configured in a substantially circular geometry, the absorbent (66) has a radius (R) which extends from a point located in the center of the absorbent to a point located on the periphery of the absorbent. The periphery of this substantially circular version may be found, as expected, along its circumference. The geometry of the absorbent (66) is a significant factor affecting the overall size and effectiveness of the absorbent article (40). In general, the absorbent (66) has a maximum radius (Rₘₐₓ) typically no greater than about 20; alternatively, no greater than about 25; alternatively, no greater than about 30; alternatively, no greater than about 35; alternatively, no greater than about 40; alternatively, no greater than about 45; or finally, alternatively, no greater than about 50 mm. The absorbent (66) may also have a minimum radius (Rₘᵢₙ) typically no less than about 45; alternatively, no less than about 40; alternatively, no less than about 35; alternatively, no less than about 30; alternatively, no less than about 25; alternatively, no less than about 20; or finally, alternatively, no less than about 15 mm. Thus, the absorbent (66) may have a radius ranging between no less than about 15 mm up to no greater than about 50 mm; although the approximate radius of the absorbent may vary according to, *inter alia*, the general design and intended disposition of the absorbent article (40) within the vestibule (42) of a female wearer.

Positioned either on or substantially parallel to the central axis (C) of certain versions of the absorbent (66), is, optionally, at least one desired axis of flexure (F). Any such desired axis of flexure (F) is generally positioned parallel to, and may be off center from, the central axis (C), a distance of no greater than about 10; alternatively, no greater than about 9; alternatively, no greater than about 8; alternatively, no greater than about 7; alternatively, no greater than about 6; alternatively, no greater than about 5; alternatively, no greater than about 4; alternatively, no greater than about 3; alternatively, no greater than about 2; or alternatively, no greater than about 1 mm. Desirably, a desired axis of flexure (F) is aligned along the central axis (C). A desired axis of flexure (F) typically minimally extends no less than about 90; alternatively, no less than about 80; alternatively, no less than about 70; alternatively, no less than about 60; alternatively, no less than about 50; or alternatively, no less than about 40 % of the length of the diameter of the absorbent (66). A desired axis of flexure (F) typically extends no greater than about 50; alternatively, no greater than about 60; alternatively, no greater than about 70; alternatively, no greater than about 80; alternatively, no greater than about 90; or alternatively, no greater than about 100 % of the length of the diameter of the absorbent (66).

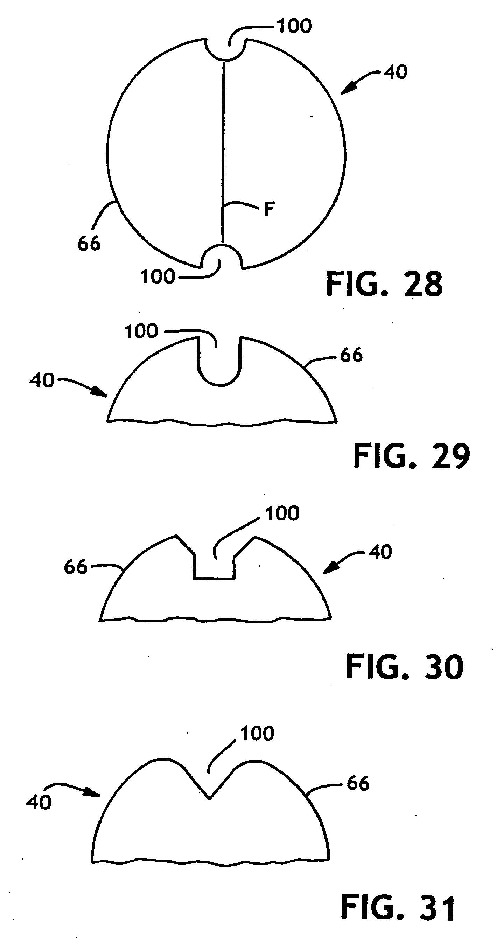

As illustrated in at least FIGs. 22, 23 and 25 through 31, an absorbent (66), and thus an absorbent articles (40), includes at least one notch (100) extending inward from the periphery of the absorbent. The notch (100) may, for example, be situated substantially on or adjacent to the central axis (C) of the absorbent (66). Alternatively, for example, the notch (100) may be situated substantially on or adjacent to a desired axis of flexure (F). The notch (100) may also provide a natural folding or bending line to the absorbent (66), thus allowing the absorbent article (40), when folded or bent along any such line or axis, to be more easily folded or bent. This is believed to be particularly true when an absorbent (66) having a substantially circular geometry, as illustrated in FIG. 28, has at least two notches (100) situated in its periphery, one notch at or near opposing ends of an axis or line. When the absorbent article (40) is disposed within the vestibule, the notch (100), when located at least in the periphery of that portion of the absorbent article to be situated nearest the clitoris (60), minimizes the likelihood that the absorbent article will irritatingly contact the sensitive clitoris.

Desirably, the notch (100) of the embodiments described herein is of dimensions sufficient to minimize the likelihood that the absorbent article (40) will, when appropriately disposed within a female wearer's vestibule (42), come into irritating contact with the clitoris (60) and/or the perineum (50), as desired. Stated differently, the notch (100) desirably is of dimensions sufficient to maximize the possibility that the absorbent article (40) will maintain a substantially spaced relationship from the clitoris (60) and/or the perineum (50), as desired, when the absorbent article is appropriately disposed within a female wearer's vestibule (42).

The notch (100) suitably extends inward from the periphery of the absorbant (66) a depth (102), as measured perpendicularly from the periphery of the absorbent, of no greater than about 30; alternatively, no greater than about 25; alternatively, no greater than about 20; alternatively, no greater than about 15; alternatively, no greater than about 10; alternatively, no greater than about 5; alternatively, no greater than about 4; or alternatively, no greater than about 3 mm. Alternatively, the notch (100) has a depth (102) of no less than about 2; alternatively, no less than about 3; alternatively, no less than about 4; alternatively, no less than about 5; alternatively, no less than about 10; alternatively, no less than about 15; alternatively, no less than about 20; or alternatively, no less than about 25 mm. Thus, the notch (100) may have a depth (102), as measured perpendicularly from the periphery of the absorbent, ranging between no less than about 2 mm up to no greater than about 30 mm; although the approximate depth of the notch may vary according to, *inter alia*, the general design and intended disposition of the absorbent article (40) within the vestibule (42) of a female wearer.

The notch (100) also has a width (104), the widest portion of which is usually situated at least on the periphery of the absorbent (66). Desirably, the notch (100) has a width (104) no greater than about 30; alternatively, no greater than about 25; alternatively, no greater than about 20; alternatively, no greater than about 15; alternatively, no greater than about 10; alternatively, no greater than about 5; alternatively, no greater than about 4; alternatively, no greater than about 3; alternatively, no greater than about 2; alternatively, no greater than about 1; or alternatively no greater than about 0.5 mm; although the approximate width of the notch may vary according to, *inter alia*, the general design and intended disposition of the absorbent article (40) within the vestibule (42) of a female wearer.

The notch (100) when configured as described herein may have a variety of geometries including U-shaped, V-shaped, W-shaped, semi-circular or a variety of combinations thereof. Several examples of possible notch (100) geometries are illustrated in several of the FIGs. One of skill in the art will recognize, however, that the notch geometries identified herein are nonlimiting and are but a few examples of the many geometries that may be suitable for the notch (100) described herein.

## Claims

1. An absorbent article (40) comprising an absorbent (66), the absorbent article being configured for disposition within the vestibule (42) of a female wearer, the absorbent having a substantially circular geometry and a periphery, the absorbent having at least one notch (100) situated in its periphery, wherein the notch extends inward from the periphery of the absorbent.

2. The absorbent article of claim 1, wherein the notch has dimensions sufficient to allow the absorbent article to maintain a substantially spaced relationship from a female wearer's clitoris (60) when the absorbent article is disposed within the vestibule.

3. The absorbent article of any preceding claim, wherein the notch has a depth (102) no greater than 30 mm; and wherein the notch has a width (104) no greater than 30 mm.

4. The absorbent article of claim 1, wherein the notch has dimensions sufficient to allow the absorbent article to maintain a substantially spaced relationship from a female weaver's perineum (50) when the absorbent article is disposed within the vestibule.

5. The absorbent article of any preceding claim, further comprising a fluid permeable cover (62).

6. The absorbent article of claim 5, wherein the cover encloses the absorbent.

7. The absorbent article of any preceding claim, further comprising a liquid impermeable baffle (64).

8. The absorbent article of any preceding claim, wherein the absorbent further comprises a superabsorbent polymer.

9. The absorbent article of any preceding claim, wherein the absorbent (66) further comprises a central axis (C) and an axis of flexure (F) substantially parallel to the central axis (C), wherein the axis of flexure (F) is coincident with the central axis (C).

10. The absorbent article of claim 9, wherein said notch (100) is situated on the axis of flexure (F).

11. The absorbent article of claim 10, wherein the absorbent comprises at least two notches (100) situated at opposing ends of the axis of flexure (F).

## Patentansprüche

1. Absorbtionsfähiger Artikel (40) mit einem Absorptionsmittel (66), wobei der absorbtionsfähige Artikel zur Anordnung innerhalb des Vestibulum (42) einer Trägerin gestaltet ist, wobei das Absorptionsmittel eine im Wesentlichen kreisförmige Geometrie und einen Umfang hat und wenigstens eine an seinem Umfang gelegene Kerbe (100) aufweist, wobei die Kerbe sich vom Umfang des Absorptionsmittels nach innen erstreckt.

2. Absorbtionsfähiger Artikel nach Anspruch 1, wobei die Kerbe Abmessungen hat, die ausreichend sind, um es dem absorbtionsfähigen Artikel zu ermöglichen, eine im Wesentlichen beabstandete Beziehung zur Klitoris (60) einer Trägerin einzuhalten, wenn sich der absorbtionsfähige Artikel innerhalb des Vestibulum befindet.

3. Absorbtionsfähiger Artikel nach einem der vorhergehenden Ansprüche, wobei die Kerbe eine Tiefe (102) von nicht mehr als 30 mm hat und eine Breite (104) von nicht mehr als 30 mm hat.

4. Absorbtionsfähiger Artikel nach Anspruch 1, wobei die Kerbe Abmessungen hat, die ausreichend sind, es dem absorbtionsfähigen Artikel zu ermöglichen, eine im Wesentlichen beabstandete Beziehung zum Perineum (50) einer Trägerin einzuhalten, wenn der absorbtionsfähige Artikel im Vestibulum angeordnet ist.

5. Absorbtionsfähiger Artikel nach einem der vorhergehenden Ansprüche, weiterhin enthaltend eine fluiddurchlässige Abdeckung (62).

6. Absorbtionsfähiger Artikel nach Anspruch 5, wobei die Abdeckung das Absorptionsmittel umschließt.

7. Absorbtionsfähiger Artikel nach einem der vorhergehenden Ansprüche, weiterhin enthaltend eine flüssigkeitsundurchlässige Sperre (64).

8. Absorbtionsfähiger Artikel nach einem der vorhergehenden Ansprüche, wobei das Absorptionsmittel weiterhin ein superabsorbtionsfähiges Polymer enthält.

9. Absorbtionsfähiger Artikel nach einem der vorhergehenden Ansprüche, wobei das Absorptionsmittel (66) weiterhin eine Mittenachse (C) und eine Biegeachse (F), die im Wesentlichen parallel zur Mittenachse (C) verläuft, hat, wobei die Biegeachse (F) mit der Mittenachse (C) zusammenfällt.

10. Absorbtionsfähiger Artikel nach Anspruch 9, wobei die Kerbe (100) in der Biegeachse (F) liegt.

11. Absorbtionsfähiger Artikel nach Anspruch 10, wobei das Absorptionsmittel wenigstens zwei Kerben (100) aufweist, die an entgegengesetzten Enden der Biegeachse (F) liegen.

## Revendications

1. Article absorbant (40) comprenant un élément absorbant (66), l'article absorbant étant configuré de manière à être disposé dans le vestibule (42) de l'utilisatrice, l'élément absorbant présentant une géométrie sensiblement circulaire et une périphérie, l'élément absorbant ayant au moins une encoche (100) située à sa périphérie, dans lequel l'encoche s'étend vers l'intérieur depuis la périphérie de l'élément absorbant.

2. Article absorbant selon la revendication 1, dans lequel l'encoche a des dimensions suffisantes pour permettre à l'article absorbant de rester en relation sensiblement espacée par rapport au clitoris (60) de l'utilisatrice lorsque l'article absorbant est disposé dans le vestibule.

3. Article absorbant selon l'une quelconque des revendications précédentes, dans lequel l'encoche a une profondeur (102) inférieure ou égale à 30 mm ; et dans lequel l'encoche a une largeur (104) inférieure ou égale à 30 mm.

4. Article absorbant selon la revendication 1, dans lequel l'encoche a des dimensions suffisantes pour permettre à l'article absorbant de rester en relation sensiblement espacée par rapport au périnée (50) de l'utilisatrice lorsque l'article absorbant est disposé dans le vestibule.

5. Article absorbant selon l'une quelconque des revendications précédentes, comprenant en outre un voile (62) perméable aux fluides.

6. Article absorbant selon la revendication 5, dans lequel le voile enveloppe l'élément absorbant.

7. Article absorbant selon l'une quelconque des revendications précédentes, comprenant en outre une couche barrière (64) imperméable aux liquides.

8. Article absorbant selon l'une quelconque des revendications précédentes, dans lequel l'élément absorbant comprend en outre un polymère superabsorbant.

9. Article absorbant selon l'une quelconque des revendications précédentes, dans lequel l'élément absorbant (66) comprend en outre un axe central (C) et un axe de flexion (F) sensiblement parallèle à l'axe central (C), l'axe de flexion (F) coïncidant avec l'axe central (C).

10. Article absorbant selon la revendication 9, dans lequel ladite encoche (100) est située sur l'axe de flexion (F).

11. Article absorbant selon la revendication 10, dans lequel l'élément absorbant comprend au moins deux encoches (100) situées aux extrémités opposées de l'axe de flexion (F).
